# EUROPÄISCHE PATENTSCHRIFT

(11) **EP 1 333 783 B1**
(45) Veröffentlichungstag und Bekanntmachung des Hinweises auf die Patenterteilung: **06.05.2004**
(21) Anmeldenummer: 01982035.6
(22) Anmeldetag: 16.11.2001
(51) Int. Cl.: A61F 2/40

(54) **ENDOPROTHESE FÜR EIN SCHULTERGELENK**
ENDOPROSTHESIS FOR A SHOULDER JOINT
ENDOPROTHESE POUR ARTICULATION DE L'EPAULE

(30) Priorität: 16.11.2000 CH 223400; 16.11.2000 CH 223500
(43) Veröffentlichungstag der Anmeldung: 13.08.2003
(73) Patentinhaber: Horber, Willi, 8005 Zürich (CH)
(72) Erfinder: Horber, Willi, 8005 Zürich (CH)
(74) Vertreter: Walder, Martin Bernhard
(86) Internationale Anmeldenummer: PCT/CH2001/000676
(87) Internationale Veröffentlichungsnummer: WO 2002/039933

(56) Entgegenhaltungen:
- EP-A- 0 024 442
- EP-A- 0 532 440
- EP-A- 0 669 117
- EP-A- 0 712 617
- EP-A- 0 808 617
- WO-A-00/01327
- WO-A-98/46172
- DE-C- 19 509 037
- DE-U- 29 918 589
- FR-A- 2 321 871
- FR-A- 2 773 469
- US-A- 3 815 157

## Beschreibung

Die Erfindung betrifft eine Endoprothese für ein Schultergelenk gemäss Oberbegriff des Patentanspruchs 1. Die Endoprothese weist ein axiales Schaftteil zum Einsetzen in den Humerus und ein mit einem Anlenkkörper am Schaftteil angelenkten Halsstück auf. Das Halsstück weist einen mit dem Halsstück richtbaren axialen Gelenkhals zur Aufnahme eines Gelenkkopfes auf. Es sind ferner Feststellmittel zum Feststellen des Halsstückes in einer gewählten Ausrichtung und ein Gelenkkopf mit einer ersten Artikulationsfläche vorhanden. Diese Artikulationsfläche wirkt mit einer zweiten künstlichen oder der natürlichen Artikulationsfläche an der Schulter zusammen. Die Prothese schliesst daher gegebenenfalls auch ein Prothesenteil mit der künstlichen zweiten Artikulationsfläche mit ein.

### Stand der Technik

Aus der EP-A-712 617 ist eine Schultergelenkprothese entsprechend der Präambel des Anspruchs 1 bekannt. Diese weist einen Schaft, der im Humeruskanal verankerbar ist, und eine Kappe mit einem im Wesentlichen halbkugelförmigen Profil auf. Die Kappe kann mit der Gelenkpfanne der Schulter zusammenwirken. Bei dieser Schultergelenkprothese weist der Schaft einen Anlenksitz mit einem kugelförmigen Boden auf. Im Anlenksitz ist eine Kugel angeordnet, die Mittel zur Aufnahme der Kappe aufweist. Zudem sind Verriegelungsmittel vorhanden, insbesondere Madenschrauben, welche die Fixierung der Kugel in einer zur Achse des Schafts bestimmten Winkelstellung ermöglichen.

Diese Schultergelenkprothese ermöglicht dank dem Kugelgelenk zwischen Schaft und Gelenkhals eine stufenlose Regelung der Inklination und der Rotation der Halsachse. Nachteilig an dieser Prothese ist, dass die Herstellung von Kugeloberflächen relativ aufwändig ist, und dass die Fixierung von Kugeloberflächen bezüglich einer konzentrischen Kugeloberfläche schwierig ist.

Die FR 2 773 469 offenbart eine Schultergelenkprothese mit Schaftteil, daran einem verschwenkbaren Richtstück, welches eine Kugelkalotte trägt. Bei dieser Prothese ist am metaphysären Schaftteilende eine hemisphärische Grube mit einer Gewindebohrung an deren Grund vorgesehen. Grube und Gewindebohrung sind auf eine Halsachse ausgerichtet, deren Winkel zur Schaftachse vorgewählt ist. In der Grube sitzt ein verschwenkbares Richtstück mit einer zur Halbkugeloberfläche der Grube konzentrischen Halbkugeloberfläche an seinem humeralen Ende. Das Richtstück besitzt eine axiale Bohrung mit einem halbkugelförmigen Grund. Eine Schraube mit Kugelkopf ist mit dem Schaft voran in die Bohrung eingeführt, wobei der Gewindeabschnitt des Schaftes durch eine konische Bohrung im halbkugelförmigen Grund des Richtstückes hindurchgestossen und in die Gewindebohrung im Schaftteil eingeschraubt ist. Die Zentren der Kugeloberflächen von Grube im Schaftteil, innerer und äusserer Halbkugeloberfläche am Richtstück und Kugeloberfläche des Schraubenkopfes liegen bei angezogener Schraube in einem gemeinsamen Zentralpunkt. Durch diese Ausbildung ist das Zwischenstück gegenüber dem Schafteil in alle Richtungen stufenlos verschwenkbar und gleichzeitig verdrehbar.

Nachteilig an dieser Prothese ist, dass die vier Kugeloberflächen ein gemeinsames Zentrum haben müssen und daher mit höchster Präzision hergestellt werden müssen, damit ein genügender Halt zwischen den Kugeloberflächen erreicht werden kann.

Aus der DE-U-29918 589.3 ist eine Schultergelenkprothese bekannt, die diese Problematik der extremen Präzision bei Kugeloberflächen vermeidet. Diese Prothese weist ein Schaftteil zum Implantieren in den Humerus mit einem Schaftkopf auf. Im Bereich des Schaftkopfs ist eine Anlenkfläche ausgebildet, an der ein Drehstück angeordnet ist. Dieses ist gegenüber dem Schaftteil um eine erste Achse verdrehbar. Am Drehstück angelenkt ist ein Richtstück, das sich entlang einer Richtachse erstreckt und bezüglich des Drehstücks um eine zweite Achse drehbar ist. Die zweite Achse verläuft quer zur ersten Achse und quer zur Richtachse. Dadurch ist die Richtachse stufenlos in alle Richtungen verschwenkbar. Drehstück und Richtstück sind jeweils in einer wählbaren Stellung fixierbar. Über das Richtstück und das Drehstück ist eine Kopfkalotte mit dem Schaftteil verbindbar.
Anlenk- oder Berührungsflächen zwischen Schaftteil und Drehstück lassen lediglich eine Relativbewegung zwischen Schaftteil und Drehstück um deren gemeinsame erste Achse zu. Anlenk- oder Berührungsflächen zwischen Drehstück und Richtstück lassen lediglich eine Relativbewegung zwischen Drehstück und Richtstück um die gemeinsame zweite Achse als Rotationszentrum zu.

Nachteilig an dieser Endoprothese ist, dass wenigstens zwei Zwischenstücke zwischen Schaftteil und Kalotte benötigt werden.

### Aufgabe der Erfindung:

Es ist deshalb Aufgabe der Erfindung, eine Schultergelenkprothese zu schaffen, bei der ebenfalls die Halsachse bezüglich Inklination und Rotation in einer wählbaren Winkelstellung zur Schaftachse feststellbar ist. Dabei soll, wie beim Kugelgelenk, der Gelenkhals über einen einzigen Anlenkkörper direkt am Schaftteil angelenkt sein. Dieser Anlenkkörper und die mit ihm zusammenwirkenden Flächen am Schaftteil sollen jedoch geringerer Präzision bedürfen als Kugeloberflächen.

### Beschreibung der Erfindung

Erfindungsgemäss wird diese Aufgabe durch die kennzeichnenden Merkmale des Patentanspruchs 1 gelöst. Erfindungsgemäss weist der Anlenkkörper wenigstens eine axialsymmetrische Anlenkfläche auf, deren Symmetrieachse senkrecht zur Gelenkhalsachse steht. Diese Anlenkfläche wirkt mit wenigstens einer Klemmfläche am Schaftteil zusammen, die zu einer Rotationsachse im Wesentlichen rotationssymmetrisch ist und deren Rotationsachse senkrecht zur Symmetrieachse der Anlenkfläche steht. Diese Klemmfläche kann insbesondere im Wesentlichen eben ausgebildet sein und zur Rotationsachse senkrecht stehen.

Vorteilhaft sind zwei gegenüberliegende Anlenkflächen axialsymmetrisch ausgebildet und wirken mit zwei lediglich rotationssymmetrischen, nicht sphärischen Klemmflächen zusammen. Es kann aber auch eine der Anlenkflächen kugelsymmetrisch ausgebildet sein und mit einer ebensolchen zusammenwirken. Es kann auch lediglich eine der zusammenwirkenden Flächen kugelsymmetrisch sein, während die andere lediglich rotationssymmetrisch ist.

Die Klemmflächen entsprechen vorteilhaft im Wesentlichen der Rotationsform des Anlenkkörpers um die Halsachse. Der Anlenkkörper besitzt vorteilhaft sowohl auf seiner zum Schaftteil hin als auch auf seiner zur Kopfkalotte hin gerichteten Seite einander gegenüberliegende Anlenkflächen. Diese können unterschiedlich ausgebildet sein, besitzen jedoch zweckmässigerweise die gleiche Symmetrieachse. Die Anlenkflächen sind vorteilhaft rotationssymmetrisch, können jedoch auch Vielflache sein, die einer Rotationsfläche angenähert sind. Einfache Formen sind im Wesentlichen zylindrisch, kegelförmig oder torusförmig. Es sind auch Mischformen dieser drei Grundformen möglich. Dabei können die Oberflächen flächig und glatt, rauh oder gerillt sein. Es können auf den Oberflächen Spitzen oder Grate ausgebildet sein. Die Anlenkflächen können auch freie Formen aufweisen, z.B. gewellt sein. Abweichungen vom einfachen Zylinder auf einer ebenen Klemmfläche haben den Vorteil, dass beim Festklemmen eine Zentrierung des Anlenkkörpers erfolgt.

Vorteilhaft sind die Anlenkflächen wenigstens in Winkelbereichen innerhalb des Schwenkwinkels des Gelenkhalses um die Symmetrieachse der Anlenkflächen rotationssymmetrisch zur Symmetrieachse oder sind Vielflache, die einer zur Symmetrieachse rationssymmetrischen Fläche angenähert sind.

Sind die Anlenkflächen durch eine Anzahl von Kanten gebildet oder weisen sie Grate, Spitze oder dergleichen auf, wird beim Festklemmen des Anlenkkörpers eine Verkrallung der Anlenkflächen mit den Klemmflächen erreicht. Ebenso können die Klemmflächen Kanten, Spitzen oder Grate aufweisen, so dass sich diese mit den Anlenkflächen verzahnen. Vorteilhaft sind an beiden zusammenwirkenden Flächen solche Kanten ausgebildet. Besonders vorteilhaft kann auch eine Verzahnung herbeigeführt werden, in dem ineinandergreifende Rillen und Grate oder Wellenstrukturen an Anlenkfläche und Klemmfläche vorgesehen sind.

Am Schaftteil sind vorteilhaft zwei einander gegenüberliegende Klemmflächen ausgebildet, zwischen denen ein Anlenkraum zur Aufnahme des Anlenkkörpers gebildet ist. Dazu weist das Schaftteil vorteilhaft einen in den Humerusknochen einsetzbaren Stiel, einen metaphysären Schaftkopf und einen auf den Schaftkopf aufsetzbaren Deckel auf. Die Klemmflächen sind am Schaftkopf und am Deckel ausgebildet. Der Deckel ist vorteilhaft aufschraubbar. Dies erlaubt das einfache Verstellen der Distanz zwischen den zwei Klemmflächen am Schaftteil und am Deckel. Es sind aber auch andere Verbindungen denkbar, z.B. Scharnier/Schraubverbindung, Einschieben des Deckels entlang einer Schiene parallel zur Klemmfläche, Aufstecken des Deckels parallel zur Rotationsachse auf den Schaftkopf und Befestigen mit quer zur Aufsteckrichtung verlaufenden Bolzen oder Schrauben oder eine integral feste Verbindung von Deckel und Schaftkopf.

Zusätzlich oder alternativ zu einem solchen ersten Klemmmittel am Schaftteil, mit welchem der Anlenkkörper zur Schaftachse hin gegen eine Klemmfläche am Schaftkopf gepresst werden kann, kann vorteilhaft ein zweites Klemmmittel vorgesehen sein. Ein zweites Klemmmittel kann aus wenigstens einer Schraube bestehen, mit der der Anlenkkörper gegenüber einer seiner Umgebungsflächen verklemmt wird. Die Schraube kann von aussen in den Anlenkraum hineingeschraubt werden.

Vorteilhaft ist jedoch durch den Anlenkkörper hindurch eine Gewindebohrung vorhanden, durch welche eine Schraube als zweites Klemmmittel schraubbar ist. Da der Gelenkhals gut zugänglich ist, liegt diese Bohrung vorteilhaft auf der Gelenkhalsachse. Die Bohrung kann lediglich durch den Anlenkkörper hindurch vorliegen, wobei dann der Gelenkhals als Schraube ausgebildet ist. Es kann in einer bevorzugten Ausführung aber auch die Gewindebohrung durch den Gelenkhals hindurchgeführt sein und eine Schraube durch ein einstückiges Halsstück mit Anlenkkörper und Gelenkhals hindurch geschraubt sein.

Vorteilhaft ist der Abstand der Klemmflächen verstellbar, um den Anlenkkörper wenigstens provisorisch zu fixieren.

Zweckmässigerweise sind am Schaftkopf und Deckel Klemmflächen oder Klemmkanten ausgebildet. Der Anlenkraum zwischen den Klemmflächen und Klemmkanten kann im Schaftkopf und/oder im Deckel ausgebildet sein.

Kurzbeschreibung der Figuren:
Anhand der schematischen und vereinfachten Figuren werden Ausführungsformen von erfindungsgemässen Schultergelenkprothesen mit vorteilhaften Merkmalen beschrieben. Es zeigt:
- Fig. 1: ein Schaftteil einer erfindungsgemässen Endoprothese mit darin angeordnetem Halsstück in einer Frontalansicht und im Anlenkbereich geschnitten,
- Fig. 2: einen Teilschnitt durch eine erfindungsgemässe Endoprothese mit drehbarer Klemmplatte und mit Exzenterring und Kopfkalotte, sowie einer Gelenkpfanne
- Fig. 3: einen Schnitt durch den Anlenkbereich einer erfindungsgemässen Endoprothese mit dem Anlenkraum in einem Deckel und konischen Anlenk - und Klemmflächen,
- Fig. 4: Aufsicht auf ein Halsstück im Anlenkraum,
- Fig. 5: Schnitt durch ein Halsstück mit einem als Klemmschraube ausgebildeten Gelenkhals,
- Fig. 6: Axialansicht eines Halsstücks mit konischen Anlenkflächen,
- Fig. 7 und 8: Seitenansicht und Axialansicht eines Halsstücks mit unten zylindrischer und oben konischer Anlenkfläche,
- Fig. 9 und 10: Seitenansicht und Axialansicht eines Halsstücks mit den Anlenkflächen unten auf dem tonnenförmig abgerundetem Gelenkhals und oben auf einem senkrecht aus dem Gelenkhals vorstehenden Bolzen,
- Fig. 11: ein Schnitt durch eine Schaftkopfpartie mit einem Halsteil, das eine zentrale Bohrung aufweist, in der der Kopf einer Befestigungsschraube sitzt.

### Beschreibung der Figuren:

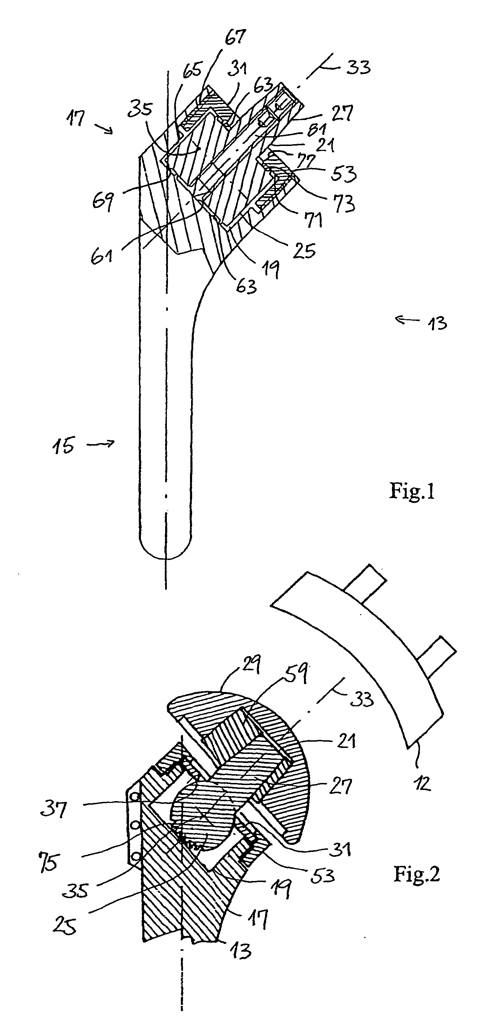

Die Figur 1 zeigt eine Endoprothese ohne Artikulationsflächen des Schultergelenks. Das Schaftteil 13 umfasst einen Stiel 15, einen Schaftkopf 17 und einen Deckel 53. Im Schaftkopf 17 ist ein Anlenkkörper 25 angelenkt, der mit dem Gelenkhals 27 zusammen ein Halsstück 21 bildet. Im Schaftkopf 17 ist eine Anlenkmulde 19 ausgebildet. Die Anlenkmulde 19 besitzt einen ebenflächigen Boden 61 mit einer Kreisrippe 63 und einer zylindrischen Wandung 65. In der Wandung 65 ist ein Innengewinde 67 eingearbeitet. In dieses Innengewinde ist ein Deckel 53 eingeschraubt. Am Deckel 53 ist eine Klemmplatte 31 ausgebildet, die zur oberen Anlenkfläche 73 des Anlenkkörpers 25 hin eine ebenflächige Klemmfläche 71 aufweist. An der Klemmfläche 71 ist eine Kreisrippe 63 ausgebildet. Der Anlenkkörper 25 ist zylindrisch und besitzt zwei Umfangrippen 69.

Durch leichtes Anziehen des Deckels 53 wird der Anlenkkörper zwischen den zwei Klemmflächen, nämlich der Klemmfläche 71 am Deckel 53 und der Bodenfläche 61 am Schaftkopf 17 eingeklemmt. Je nach Klemmkraft kann das Halsstück 21 noch um die Schwenkachse 35, die die Symmetrieachse der Anlenkflächen ist, verschwenkt und um die Achse 33 der Anlenkmulde 19 rotiert werden. In der Klemmplatte 31 ist eine kreisrunde, konische zentrale Öffnung 77 vorhanden. Durch diese Öffnung 77 hindurch reicht der Gelenkhals 27 aus der Anlenkmulde 19 heraus. Die Öffnung 77 ist derart bemessen, dass der Gelenkhals 27 in ausreichendem Mass in jede Richtung verschwenkbar ist.

Durch den Gelenkhals 27 hindurch ist eine Bohrung mit einem Innengewinde vorhanden, in die eine Madenschraube 81mit einer scharfen Spitze eingeschraubt ist.

Durch Einschrauben der Madenschraube kann die Spitze gegen den Boden 61 geschraubt und in diesen hineingegraben werden. Dadurch erfolgt eine definitive Fixierung des Halsstückes 21 am Schaftteil 13. Zur Sicherung der Madenschraube 81 ist eine zweite Schraube ins Gewinde eingeschraubt und gegen die Madenschraube gepresst.

In Figur 2 ist ein zweites Ausführungsbeispiel dargestellt, dessen Anlenkkörper 25 ebenfalls eine zylindrische Form hat. Die Anlenkmulde 19 ist auch hier zylindrisch. Die Zylinderachse des Anlenkkörpers 25 verläuft senkrecht zur Halsachse 33, während die Zylinderachse der Anlenkmulde 19 senkrecht zur Zylinderachse des Anlenkkörpers 19 steht. Der Grund der Anlenkmulde 19 ist eben und senkrecht zu ihrer Zylinderachse.

Der Anlenkkörper 25 ist gerade so lang, dass die Kanten seiner Stirnseiten die Wand der Anlenkmulde 19 berühren ohne zu klemmen und der Anlenkkörper stets zentriert in der Anlenkmulde verdrehbar ist.

Diese Drehung macht die Klemmplatte 31 jeweils mit. Sie liegt mit einer geraden Öffnungskante 37 an der Zylinderoberfläche an und ist kreisrund. Die Klemmplatte 31 ist durch eine Überwurfmutter 53 am Schaftkopf 17 befestigt und ist zwischen diesen frei verdrehbar. Dadurch kann die stufenlos einstellbare Abweichung der Halsachse 33 von der Zylinderachse der Anlenkmulde 19 stufenlos um 360 Grad senkrecht auf die Ebene der Knochenschnittfläche ausgerichtet werden. Dies gibt dieselbe kugelgelenkartige Freiheit der Ausrichtung der Halsachse wie ein echtes Kugelgelenk. Die dem Grund der Anlenkmulde 19 zugerichtete Anlenkfläche 75 des Anlenkkörpers 25 ist gerillt, so dass Kanten auf der Zylinderoberfläche ausgebildet sind, die sich beim Anziehen der Überwurfmutter 53 in den ebenen Grund der Anlenkmulde 19 Furchen einpressen. Durch diese Verkrallung von Anlenkmulde und Anlenkkörper 25 ist sowohl die Verdrehung um die Zylinderachse der Anlenkmulde 19 als auch um die Zylinderachse 35 des Anlenkkörpers 25 blockiert. Bei diesem Ausführungsbeispiel kann die Klemmplatte auch von der Seite her zwischen die Überwurfmutter 53 und den Anlenkkopf einschiebbar sein. Dazu benötigt die Überwurfmutter kein Gewinde, sondern kann z.B. in eine um die Anlenkmulde umlaufende Nut eingreifend ausgebildet sein. Bei einer Keilform der Klemmplatte und einer entsprechend winkligen Berührungsfläche an der Überwurfmutter kann die Pressung auf den Anlenkkopf durch Einschlagen der Klemmplatte erreicht werden.

In Figur 2 ist weiter ein auf dem Gelenkhals 27 aufgesteckter Exzenterring 59 dargestellt. Der Exzenterring 59 kann für die Einstellung der Exzentrizität der Kalotte 29 bezüglich Gelenkhals 27 um die Gelenkhalsachse verdreht werden. Weiter ist eine künstliche Gelenkpfanne 12 dargestellt.

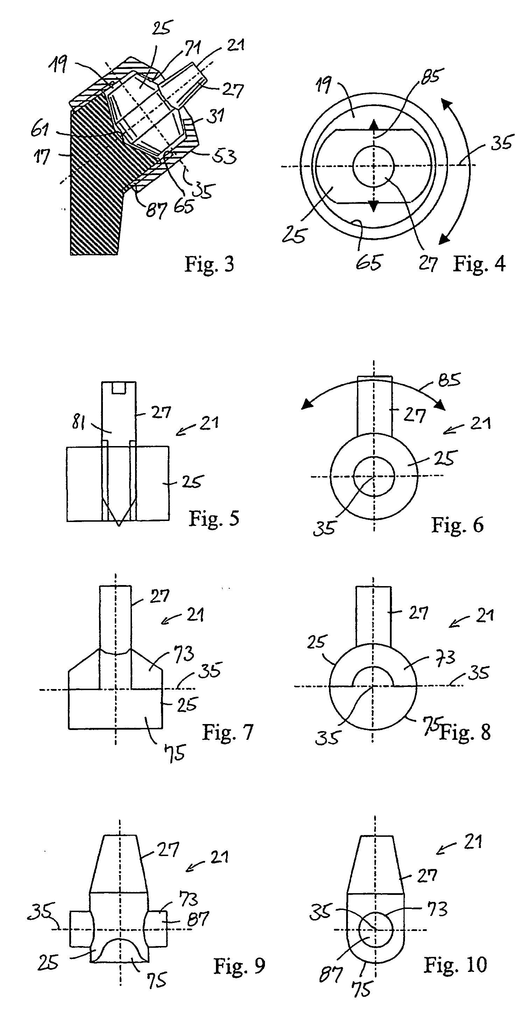

In Figur 3 ist ein drittes Ausführungsbeispiel dargestellt. Der Anlenkraum 19 ist im Deckel 53 ausgebildet, der auf ein am Schaftkopf 17 ausgebildetes Aussengewinde 87 aufgeschraubt ist. Der Anlenkkörper 25 besitzt zwei entgegengesetzt gerichtete Kegelstumpfflächen als Anlenkflächen. Der Boden 61 des Anlenkraumes 19 ist ebenfalls kegelstumpfförmig konkav ausgebildet. Die beiden Winkel der zusammenwirkenden Kegelmantelflächen entsprechen einander komplementär. Die Klemmplatte 31 am Deckel 53 besitzt ebenfalls eine kegelstumpfförmige Klemmfläche 71.

Die Klemmplatte 31 kann auch als Unterlagsscheibe ausgebildet sein, die mit einer Überwurfmutter 53 gegen den Anlenkkörper 25 gepresst wird. Sie muss daher nicht einstückig mit der Überwurfmutter ausgebildet sein. Dies hat den Vorteil, dass beim Anziehen der Überwurfmutter 53 eine Verdrehung der Überwurfmutter sich weniger auf den Anlenkkörper 25 überträgt. Die Klemmflächen des Bodens 61 und/oder der Klemmplatte 31 können auch als eine oder mehrere komplementär kegelstumpfförmig angeordnete ringförmige Kanten ausgebildet sein.

In Figur 4 ist dargestellt, wie der Anlenkkörper 25 in die zylindrische Wandung 65 des Anlenkraumes 19 eingepasst ist. Die axialen Enden des Anlenkkörpers 25 sind etwa konzentrisch zur Wandung 65 konvex gewölbt ausgebildet. Damit eine Verschwenkung (Pfeil 85) des Anlenkkörpers 25 um die Schwenkachse 35 möglich ist, muss ein Abstand zwischen der Wandung 65 und dem Anlenkkörper 25 eingehalten werden. Da normalerweise etwa 10 Grad Verschwenkbarkeit ausreichend sind, kann der Abstand gering sein.

Figur 5 zeigt die grundsätzliche Möglichkeit, den Gelenkhals 27 direkt als Feststellschraube auszubilden. Im Anlenkkörper 25 ist eine Gewindebohrung vorhanden, in der der Gelenkhals eingeschraubt ist. Am Gelenkhals ist eine Spitze ausgebildet, die durch Anziehen der Schraube in den Boden 61 eines Anlenkraumes eingrabbar ist. Der Boden 61 kann in dem entsprechenden Bereich eine Kugeloberfläche aufweisen, damit die Spitze immer senkrecht auf die Bodenoberfläche auftrifft.

Figur 6 zeigt die Verschwenkbarkeit des Halsstücks 25 (Doppelpfeil 85). Dargestellt ist ein Halsstück wie in Figur 3, dessen Anlenkkörper beidseitig des Gelenkhalses 27 je eine kegelstumpfförmige Anlenkfläche aufweist.

Die Anlenkflächen müssen oben und unten nicht gleich sein. In Figuren 7 und 8 ist ein Halsstück 21 dargestellt, dessen Anlenkkörper 25 oben jeweils eine konische Anlenkfläche 73 und unten eine zylindrische Anlenkfläche 75 aufweist.

Es ist möglich, dass der Gelenkhals 27 selber einen Teil des Anlenkkörpers 25 bildet. In Figuren 9 und 10 ist ein solches Halsstück 21 dargestellt, bei dem die Unterseite tonnenförmig gerundet ist und eine Anlenkfläche 75 bildet. Die obere Anlenkfläche 73 ist auf einem den Gelenkhals 27 querenden Bolzen 87 ausgebildet.

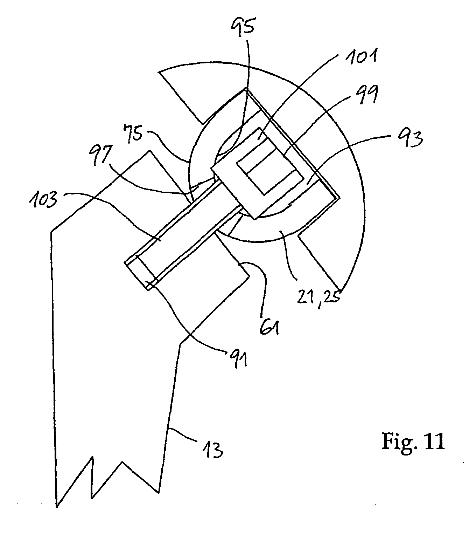

Es besteht auch die Möglichkeit, das Halsteil mittels einer zentralen Schraube im Schaftteil zu befestigen. In Figur 11 ist ein Schaftteil 13 dargestellt, welches eine ebene oder z.B. durch Kanten, Spitzen oder ähnliches strukturierte Klemmfläche 61 aufweist. Zentral in der Klemmfläche 61 ist eine Gewindebohrung 91 vorhanden.

Auf der Klemmfläche 61 liegt eine zylindrische Anlenkfläche 75 des Halsteils 21 auf. Das Halsteil 21 besitzt quer zur Halsachse einen kreisrunden Querschnitt. Konzentrisch zum Umfang ist darin eine Hohlraum-Bohrung 93 mit einem halbkugeligen Grund 95 eingearbeitet. Im Grund ist eine nach aussen sich öffnende, konische Öffnung 97 vorhanden. Eine Schraube 99 sitzt mit einem Kopf 101 in der Hohlraumbohrung 93 und ihr Gewindeschaft 103 erstreckt sich durch die Öffnung 97 im Grund 95 der Hohlraumbohrung 93 in die Gewindebohrung 91 im Schaftteil 13. Der Schraubenkopf 101 besitzt eine Kreiskante, die beim Anziehen der Schraube 99 an den halbkugeligen Grund anliegt und sich in den Grund eingräbt. Durch die Anpresskraft verformt sich entweder die Klemmfläche 61 oder die Anlenkfläche 75, so dass eine Rotation des Halsteils 21 um seine Halsachse verhindert ist. Ein Schwenken des Halsteils um die Symmetrieachse der zylindrischen Anlenkfläche 75 ist durch die Verbindung zwischen Schraubenkopf 101 und Halsteil 21 verhindert. Die Symmetrieachse der Anlenkfläche 75 geht durch das Zentrum der Kugeloberfläche des Grundes 95.

## Patentansprüche

1. Endoprothese für ein Schultergelenk, mit einer im Glenoid des Schulterblattes verankerbaren Gelenkpfanne (12) oder zum gelenkigen Zusammenwirken mit dem natürlichen Glenoid, einem Schaftteil (13) mit einem Stiel (15) und einem metaphysären Schaftkopf (17) zum Einsetzen in den Humerus, einem mit einem Anlenkkörper (25) am Schaftkopf (17) angelenkten Halsstück (21), welches einen mit dem Halsstück (21) richtbaren axialen Gelenkhals (27) zur Aufnahme einer Kopfkalotte (29) aufweist, Feststellmitteln (53,81) zum Feststellen des Halsstückes (21) in einer gewählten Ausrichtung, und einer Kopfkalotte (29) zur Artikulation mit der Gelenkpfanne (12) oder dem natürlichen Glenoid,
**dadurch gekennzeichnet,**
- **dass** der Anlenkkörper (25) eine lediglich axialsymmetrische Anlenkfläche (73,75) aufweist, deren Symmetrieachse (35) senkrecht zur Gelenkhalsachse steht,
- und **dass** die Anlenkfläche (73,75) mit wenigstens einer zu einer Rotationsachse (33) im Wesentlichen rotationssymmetrischen und/oder einer im Wesentlichen ebenen und zur Rotationsachse senkrecht stehenden Klemmfläche (71,61) am Schaftteil (13) zusammenwirkt, deren Rotationsachse (33) senkrecht zur Symmetrieachse (35) der Anlenkfläche (73,75) steht.

2. Endoprothese nach Anspruch 1, **dadurch gekennzeichnet, dass** die Anlenkfläche (75,73) in Winkelbereichen innerhalb des Schwenkwinkels des Gelenkhalses (27) um die Symmetrieachse (35) der Anlenkfläche (75,73) rotationssymmetrisch zur Symmetrieachse (35) ist.

3. Endoprothese nach Anspruch 1 oder 2, **dadurch gekennzeichnet, dass** die Anlenkfläche (75,73) in Winkelbereichen innerhalb des Schwenkwinkels des Gelenkhalses um die Symmetrieachse der Anlenkfläche (75,73) ein Vielflach ist, das einer zur Symmetrieachse rationssymmetrischen Fläche angenähert ist.

4. Endoprothese nach einem der vorangehenden Ansprüche, **dadurch gekennzeichnet, dass** die Anlenkfläche (75,73) durch eine Anzahl von Kanten gebildet ist.

5. Endoprothese nach einem der vorangehenden Ansprüche, **dadurch gekennzeichnet, dass** einander in Gelenkhalsachsrichtung gegenüberliegende Anlenkflächen (75,73) des Anlenkkörpers (25) unterschiedlich ausgebildet sind.

6. Endoprothese nach einem der Ansprüche 1 bis 5, **dadurch gekennzeichnet, dass** wenigstens eine Anlenkfläche (75,73) im Wesentlichen eine Kegelform aufweist.

7. Endoprothese nach einem der Ansprüche 1 bis 6, **dadurch gekennzeichnet, dass** wenigstens eine Anlenkfläche (75,73) im Wesentlichen eine Zylinderform aufweist.

8. Endoprothese nach einem der Ansprüche 1 bis 7, **dadurch gekennzeichnet, dass** wenigstens eine Anlenkfläche (75,73) im Wesentlichen eine Torusform aufweist.

9. Endoprothese nach einem der Ansprüche 1 bis 8, **dadurch gekennzeichnet, dass** wenigstens eine Anlenkfläche (75,73) eine gewellte Oberfläche aufweist.

10. Endoprothese nach einem der Ansprüche 1 bis 9, **dadurch gekennzeichnet, dass** das Schaftteil (13) ein erstes Klemmmittel (53,99) aufweist, mit welchem der Anlenkkörper (25) gegen das Schaftteil (13) gepresst werden kann.

11. Endoprothese nach einem der Ansprüche 1 bis 10, **dadurch gekennzeichnet, dass** durch den Anlenkkörper (25) hindurch eine Gewindebohrung vorhanden ist, durch welche eine Schraube (81) als zweites Klemmmittel schraubbar ist.

12. Endoprothese nach Anspruch 11, **dadurch gekennzeichnet, dass** der Gelenkhals (27) das zweite Klemmmittel bildet.

13. Endoprothese nach Anspruch 11, **dadurch gekennzeichnet, dass** die Gewindebohrung durch den Gelenkhals (27) hindurchgeführt ist.

14. Endoprothese nach einem der Ansprüche 1 bis 13, **dadurch gekennzeichnet, dass** am Schaftteil (13) zwei einander gegenüberliegende Klemmflächen (71,61) ausgebildet sind, zwischen denen ein Anlenkraum (19) zur Aufnahme des Anlenkkörpers (25) gebildet ist.

15. Endoprothese nach Anspruch 14, **dadurch gekennzeichnet, dass** der Abstand der Klemmflächen (71,61) verstellbar ist.

16. Endoprothese nach einem der Ansprüche 1 bis 15, **dadurch gekennzeichnet, dass** das Schaftteil (13) einen auf den Schaftkopf (17) aufschraubbaren Deckel (53) aufweist.

17. Endoprothese nach Anspruch 16, **dadurch gekennzeichnet, dass** der Anlenkraum (19) im Schaftkopf (17) ausgebildet ist.

18. Endoprothese nach Anspruch 16 oder 17, **dadurch gekennzeichnet, dass** der Anlenkraum (19) im Deckel (53) ausgebildet ist.

## Claims

1. An endoprosthesis for a shoulder joint, with a joint socket (12) for anchoring in the shoulder blade glenoid or for pivotal co-operation with the natural glenoid, a shaft piece (13) with a shank (15) and a metaphysal shaft head (17) for insertion into the humerus, a neck piece (21) articulated with an articulation member (25) on the shaft head (17) and comprising an axial joint neck (27) adapted to be aligned with the neck piece (21) and to receive a head cap (29), fixing means (53, 81) for fixing the neck piece (21) in a selected alignment, and a head cap (29) for articulation with the joint socket (12) or the natural glenoid,
**characterized in that**
- the articulation member (25) has an articulation surface (73, 75) which is only axial symmetrical and the axis of symmetry (35) of which is perpendicular to the joint neck axis,
- and **in that** the articulation surface (73, 75) co-operates with at least one clamping surface (71, 61) on the shaft piece (13), said clamping surface being substantially rotationally symmetrical to a rotational axis (33) and/or being substantially planar and perpendicular to the rotational axis, the rotational axis (33) being perpendicular to the axis of symmetry (35) of the articulation surface (73, 75).

2. An endoprosthesis according to claim 1, **characterized in that** the articulation surface (75, 73) is rotationally symmetrical to the axis of symmetry (35) in angular zones within the pivoting angle of the joint neck (27) about the-axis of symmetry (35) of the articulation surface (75, 73).

3. An endoprosthesis according to claim 1 or 2, **characterized in that** in angular zones within the pivoting angle of the joint neck about the axis of symmetry of the articulation surface (75, 73), the articulation surface (75, 73) is a polyhedron approximating a surface rotationally symmetrical to the axis of symmetry.

4. An endoprosthesis according to any one of the preceding claims, **characterized in that** the articulation surface (75, 73) is formed by a number of edges.

5. An endoprosthesis according to any one of the preceding claims, **characterized in that** articulation surfaces (75, 73) of the articulation member (25) which are situated opposite one another in the direction of the joint neck axis are formed differently.

6. An endoprosthesis according to any one of claims 1 to 5, **characterized in that** at least one articulation surface (75, 73) has substantially a conical shape.

7. An endoprosthesis according to any one of claims 1 to 6, **characterized in that** at least one articulation surface (75, 73) has substantially a cylindrical shape.

8. An endoprosthesis according to any one of claims 1 to 7, **characterized in that** at least one articulation surface (75, 73) has substantially a toroidal shape.

9. An endoprosthesis according to any one of claims 1 to 8, **characterized in that** at least one articulation surface (75, 73) has a corrugated surface.

10. An endoprosthesis according to any one of claims 1 to 9, **characterized in that** the shaft piece (13) has a first clamping means (53, 99) by which the articulation member (25) can be pressed against the shaft piece (13).

11. An endoprosthesis according to any one of claims 1 to 10, **characterized in that** a screwthreaded bore extends through the articulation member (25) and a screw (81) can be screwed therethrough as a second clamping means.

12. An endoprosthesis according to claim 11, **characterized in that** the joint neck (27) forms the second clamping means.

13. An endoprosthesis according to claim 11, **characterised in that** the screwthreaded bore extends through the joint neck (27).

14. An endoprosthesis according to any one of claims 1 to 13, **characterized in that** two oppositely situated clamping surfaces (71, 61) are formed on the shaft piece (13) and between them an articulation chamber (19) is formed to receive the articulation member (28).

15. An endoprosthesis according to claim 14, **characterized in that** the distance between the clamping surfaces (71, 61) is adjustable.

16. An endoprosthesis according to any one of claims 1 to 15,' **characterised in that** the shaft piece (13) has a cover (53) screwable on the shaft head (17).

17. An endoprosthesis according to claim 16, **characterized in that** the articulation chamber (19) is formed in the shaft head (17).

18. An endoprosthesis according to claim 16 or 17, **characterized in that** the articulation chamber (19) is formed in the cover (53).

## Revendications

1. Endoprothèse pour une articulation scapulo-humérale avec une cavité articulaire (12) pouvant être ancrée dans la cavité glénoïde de l'omoplate ou pour l'action conjointe articulée avec la cavité glénoïde naturelle, une partie tige (13) avec un manche (15) et une tête de tige métaphysaire (17) pour mettre en place dans l'humérus, une pièce de col (21) articulée avec un corps d'articulation (25) sur la tête de tige (17), pièce de col qui présente un col d'articulation (27) axial pouvant être orienté avec la pièce de col pour loger une calotte de tête (29), des moyens de blocage (53, 81) pour bloquer la pièce de col (21) dans une -orientation choisie et une calotte de tête (29) pour l'articulation avec la cavité articulaire (12) ou avec la cavité glénoïde naturelle,
**caractérisée en ce**
- **que** le corps d'articulation (25) présente une surface d'articulation (73, 75) seulement à symétrie axiale dont l'axe de symétrie (35) est perpendiculaire à l'axe du col de l'articulation
- et **que** la surface d'articulation (73, 75) a une action conjointe sur la partie tige (13) avec au moins une surface de serrage (71, 61) qui est substantiellement de rotation symétrique avec un axe de rotation (33) et/ou substantiellement plane et perpendiculaire à l'axe de rotation, surface de serrage dont l'axe de rotation (33) est perpendiculaire à l'axe de symétrie (35) de la surface d'articulation (73, 75).

2. Endoprothèse selon la revendication 1, **caractérisée en ce que** la surface d'articulation (75, 73) est de rotation symétrique à l'axe de symétrie (35) dans des zones angulaires à l'intérieur de l'angle de pivotement du col de l'articulation (27) autour de l'axe de symétrie (35) de la surface d'articulation (75, 73).

3. Endoprothèse selon la revendication 1 ou 2, **caractérisée en ce que** la surface d'articulation (75, 73) est un polyèdre dans des zones angulaires à l'intérieur de l'angle de pivotement du col de l'articulation autour de l'axe de symétrie de la surface d'articulation (75, 73), polyèdre qui est approché d'une surface de rotation symétrique à l'axe de symétrie.

4. Endoprothèse selon l'une des revendications précédentes, **caractérisée en ce que** la surface d'articulation (75, 73) est formée par un certain nombre d'arêtes.

5. Endoprothèse selon l'une des revendications précédentes, **caractérisée en ce que** des surfaces d'articulation (75, 73) du corps d'articulation (25) qui sont opposées l'une à l'autre dans le sens de l'axe du col d'articulation sont configurées différemment.

6. Endoprothèse selon l'une des revendications 1 à 5, **caractérisée en ce qu'**au moins une surface d'articulation (75, 73) présente substantiellement une forme conique.

7. Endoprothèse selon l'une des revendications 1 à 6, **caractérisée en ce qu'**au moins une surface d'articulation (75, 73) présente substantiellement une forme cylindrique.

8. Endoprothèse selon l'une des revendications 1 à 7, **caractérisée en ce qu'**au moins une surface d'articulation (75, 73) présente substantiellement une forme torique.

9. Endoprothèse selon l'une des revendications 1 à 8, **caractérisée en ce qu'**au moins une surface d'articulation (75, 73) présente une surface ondulée.

10. Endoprothèse selon l'une des revendications 1 à 9, **caractérisée en ce que** la partie tige (13) présente un premier moyen de serrage (53, 99) avec lequel le corps d'articulation (25) peut être pressé contre la partie tige (13).

11. Endoprothèse selon l'une des revendications 1 à 10, **caractérisée en ce qu'**il existe une forure filetée à travers le corps d'articulation (25) à travers laquelle une vis (81) peut être vissée comme second moyen de serrage.

12. Endoprothèse selon la revendication 11, **caractérisée en ce que** le col d'articulation (27) forme le second moyen de serrage.

13. Endoprothèse selon la revendication 11, **caractérisée en ce que** la forure filetée traverse le col d'articulation (27).

14. Endoprothèse selon l'une des revendications 1 à 13, **caractérisée en ce que** deux surfaces de serrage (71, 61) opposées l'une à l'autre sont configurées sur la partie tige (13), surfaces de serrage entre lesquelles un espace d'articulation (19) est formé pour loger le corps d'articulation (25).

15. Endoprothèse selon la revendication 14, **caractérisée en ce que** l'écart entre les surfaces de serrage (71, 61) est réglable.

16. Endoprothèse selon l'une des revendications 1 à 15, **caractérisée en ce que** la partie tige (13) présente un couvercle (53) pouvant être vissé sur la tête de tige (17).

17. Endoprothèse selon la revendication 16, **caractérisée en ce que** l'espace d'articulation (19) est configuré dans la tête de tige (17).

18. Endoprothèse selon la revendication 16 ou 17, **caractérisée en ce que** l'espace d'articulation (19) est configuré dans le couvercle (53).
